(19) Europäisches Patentamt
European Patent Office
Office européen des brevets

(11) **EP 2 376 125 B1**

(12) **EUROPEAN PATENT SPECIFICATION**

(45) Date of publication and mention
of the grant of the patent:
**31.08.2016 Bulletin 2016/35**

(51) Int Cl.:
*A61K 41/00* (2006.01)     *A61K 47/48* (2006.01)
*A61K 49/18* (2006.01)     *C12N 5/078* (2010.01)

(21) Application number: **09799734.0**

(22) Date of filing: **18.12.2009**

(86) International application number:
**PCT/IB2009/055849**

(87) International publication number:
**WO 2010/070620 (24.06.2010 Gazette 2010/25)**

(54) **ERYTHROCYTE-BASED DELIVERY SYSTEM, METHOD OF PREPARATION AND USES THEREOF**

ABGABESYSTEM AUF ERYTHROZYTENBASIS, HERSTELLUNGSVERFAHREN UND IHRE VERWENDUNGEN

SYSTÈME D ADMINISTRATION DÉLIVRANCE À BASE D'ÉRYTHROCYTE, PROCÉDÉ DE PRÉPARATION ET UTILISATIONS DE CELUI-CI

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **21.12.2008 US 139617 P**

(43) Date of publication of application:
**19.10.2011 Bulletin 2011/42**

(73) Proprietor: **Consiglio Nazionale Delle Ricerche 00185 Roma (IT)**

(72) Inventors:
• **LISI, Antonella**
  **I-00133 Roma (IT)**
• **CINTI, Caterina**
  **I-53100 Siena (IT)**
• **GRIMALDI, Settimio**
  **I-00133 Roma (IT)**

(74) Representative: **Capasso, Olga**
**De Simone & Partners SpA**
**Via Vincenzo Bellini, 20**
**00198 Roma (IT)**

(56) References cited:
**WO-A2-2008/003524     US-A1- 2007 243 137**

• **ROSSI L ET AL: "Erythrocyte-based drug delivery" EXPERT OPINION ON DRUG DELIVERY, ASHLEY PUBLICATIONS, GB, vol. 2, no. 2, 1 January 2005 (2005-01-01) , pages 311-322, XP009103048 ISSN: 1742-5247 cited in the application**
• **BRAHLER M ET AL: "Magnetite-loaded carrier erythrocytes as contrast agents for magnetic resonance imaging" NANO LETTERS, ACS, WASHINGTON, DC, US, vol. 6, no. 11, 1 November 2006 (2006-11-01), pages 2505-2509, XP008088205 ISSN: 1530-6984**
• **KHOSHNEJAD M ET AL: "Modified influenza virosomes: Recent advances and potential in gene delivery" CURRENT MEDICINAL CHEMISTRY, vol. 14, no. 29, 2007, pages 3152-3156, XP002572304 ISSN: 0929-8673**
• **GUTIERREZ MILLAN C ET AL: "In vitro studies of amikacin-loaded human carrier erythrocytes" TRANSLATIONAL RESEARCH, ELSEVIER, AMSTERDAM, NL, vol. 152, no. 2, 1 August 2008 (2008-08-01), pages 59-66, XP023314152 ISSN: 1931-5244 [retrieved on 2008-06-26] cited in the application**
• **YEZHELYEV M V ET AL: "Emerging use of nanoparticles in diagnosis and treatment of breast cancer" LANCET ONCOLOGY, LANCET PUBLISHING GROUP, LONDON, GB, vol. 7, no. 8, 1 August 2006 (2006-08-01), pages 657-667, XP025228942 ISSN: 1470-2045 [retrieved on 2006-08-01] cited in the application**

## Description

### Field of the invention

The invention relates generally to a new cell-based drug delivery system and more specifically to "erythro-magneto-virosomes" which have the capacity to encapsulate therapeutic compounds with different biological, pharmacological and chemical characteristics, such as anti-retroviral compounds, small molecules, phosphorylated pro-drugs or chemotherapeutics. Such system is also able to specifically drive the pro-drug at the target tissues or organs by application of specific electromagnetic fields at cyclotron resonance of iron ions. The invention concerns also the method of preparation and uses of the cell-based system in particular as delivery system.

### Background art

Drug delivery has been greatly improved over the years by means of chemical and physical agents that increase bioavailability, improve pharmacokinetic and reduce toxicities. At the same time, cell based delivery systems have also been developed. The use of cells as therapeutic carriers has increased in the past few years and has developed as a distinct concept and delivery method. Cell-based vehicles are particularly attractive for delivery of bio-therapeutic agents that are difficult to synthesize, have reduced half-lives, limited tissue penetrance or are rapidly inactivated upon direct in vivo introduction. As a matter of facts, the cell-based delivery system possesses a number of advantages including prolonged delivery times, targeting of drugs to specialized cell compartments and biocompatibility. The use of a physiological carrier to deliver therapeutics throughout the body to both improve their efficacy while minimising inevitable adverse side effects, is an appealing perspective that can be applied in many clinical settings.

The behaviour of erythrocytes, as a delivery system for several classes of molecules (i.e., proteins, including enzymes and peptides, therapeutic agents in the form of nucleotide analogues, gluco-corticoid analogues), has been studied extensively as they possess several properties which make them unique and useful carriers. Furthermore, the possibility of using carrier erythrocytes for selective drug targeting to differentiate macrophages increases the opportunities to treat intracellular pathogens and to develop new drugs. Finally, the availability of an apparatus that permits the encapsulation of drugs into autologous erythrocytes has made this technology available in many clinical settings and competitive in respect to other drug delivery systems.

The erythrocyte-based drug delivery presents the following advantages in that:

    i) it is especially efficient for releasing drugs in the circulation for long period of time (weeks);
    ii) erythrocytes have a large encapsulating capacity;
    iii) erythrocytes can be easily processed and can accommodate traditional and biologic drugs.

These carriers have also been used for delivering antigens and/or contrasting agents. Carrier erythrocytes have been evaluated in thousands of drug administration in humans proving safety and efficacy of the treatments. Erythrocyte-based delivery of new and conventional drugs is thus experiencing increasing interests in drug delivery and in managing complex pathologies especially when side effects could become serious issues (Pierigè F et al 2008; Rossi L et al 2005; Roth et al 2008). Recently, erythrocytes have been used to encapsulate an antibiotic, the amikacin, and a sustained release from loaded erythrocytes over a 48 hours period was demonstrated, suggesting a potential use of the erythrocytes as a slow systemic-release system for antibiotics. The administration to rats of amikacin encapsulated in erythrocytes leads to significant changes in the pharmacokinetic behaviour of the antibiotic. Indeed, a greater drug accumulation is observed in the reticulo-endothelial system (RES) of organs such as liver and spleen. This shows that loaded erythrocytes are potentially useful for the delivery of antibiotics in phagocytic cells located in the RES (Gutierrez-Millan et al. 2007; Gutierrez-Millan et al. 2008).

However, one important aspect of cell-based delivery yet to be fully investigated is the process of actual delivery of cell payload, i.e which is actually transported *in vivo.* In this regard, the potential ability of cell carriers to provide site-specific or targeted delivery of therapeutics has yet to be fully explored. Development of cell targeting strategies will further advance cell vehicle applications, broaden the applicability of this delivery approach and potentiate therapeutic outcomes.

In recent years, biomedical research indicated that magnetic nano-particles can be a promising tool for several applications in vitro and in vivo. In medicine, many approaches were investigated for diagnosis and therapy, offering a great variety of applications. Super-paramagnetic and magnetic nano-particles (magnetite-maghemite) are currently used as contrast agents for magnetic resonance imaging. Magnetic cell separation, magnetic resonance imaging (MRI), magnetic targeted delivery of therapeutics or magnetically induced hyperthermia are approaches of particular clinical relevance. For medical use, especially for *in vivo* application it is of great importance that these particles do not have any toxic effects or incompatibility with the biological organism. Investigations on applicable particles induced a variability of micro- and nanostructures with different materials, sizes, and specific surface chemistry (Alexiou C et al. 2006a). These particles can also be used as drug carriers for local chemotherapy, called magnetic drug targeting. Using an external magnetic field, colloidal nano-particles can be directed to a specific body compartment (i.e. tumor). The biodistribution of magnetic nano-particles can be visualized with X-ray imaging and then confirmed by histological analysis (Alexiou C et al. 2007). Magnetic drug targeting employing nano-particles as carriers is a promising cancer treatment avoiding side effects of conventional chemotherapy (Alexiou C et al. 2006b). The biological application of nano-particles is a rapidly developing area of the nanotechnology field that raises new possibilities in the diagnosis and treatment of human cancers. In cancer diagnostics, fluorescent nano-particles can be used for multiplex simultaneous profiling of tumour biomarkers and for detection of multiple genes and matrix RNA with fluorescent in-situ hybridisation. Supermagnetic nano-particles have exciting potentials as contrast agents for cancer detection in vivo, and for monitoring the response to treatment. Several chemotherapy agents are available as nano-particle formulations. Such formulations have at least equivalent efficacy and fewer toxic effects compared with conventional formulations. Ultimately, the use of nano-particles should allow a unique and simultaneous tumour targeting and drug delivery (Yezhelyev MV et al. 2006).

The present invention relates magnetic nano-particles and/or erythrocytes that may be transformed, chemically modified and compositions comprising them. In particular erythro-magneto-virosomes were designed. Such systems can act as bioactive pro-drug carriers, containing phosphorylated and or non phosphorylated compounds, which do not naturally occur in a human or animal organism, said compounds having different biological and/or pharmacological properties and chemical characteristics. The delivery system of the invention can be applied in many clinical settings and are useful, for example, for the treatment of neoplastic pathologies or pathologies caused by the infection of a human or animal virus.

To develop a targeted cell-based drug delivery system, the authors have produced erythro-magneto-virosomes. Such system is able to specifically drive a pro-drug at its target tissue or organ, having a high efficient fusion capability with the targeted cells. In the present invention, erythrocytes were engineered with both novel targeting proteins, also called fusion proteins and nano-particles.

The targeting proteins, in particular from viral origin, improve the integration of the system with the cytoplasmatic membranes of target cells. Such proteins are able to induce the fusion of the membrane of the delivery system to the membrane of the target cell. Furthermore, by applying physical methods, it is possible to control the distribution and location of the system thanks to the nano-particles. In the present invention, a targeting system using chemically modified nano-particles to carry the pro-drug to the active site was also developed.

The "erythro-magneto-virosomes", are engineered erythrocytes comprising paramagnetic or super-paramagnetic nano-particles and fusion-proteins. In particular, the nano-particles are inside the cell while the fusion-proteins are located on the cytoplasmic membrane. Fusion-proteins are preferably virosome fusion-proteins. Pro-drugs can be inserted into such engineered erythrocytes therefore functioning as drug delivery system. Pro-drugs, especially in their phosphorylated forms, remain stable in the engineered erythrocytes until they reach their target cells. The target cells are able to integrate the phosphorylated compounds.

Surprisingly, the erythro-magneto-virosome delivery system of the invention has a high fusion efficiency with the cytoplasmic membranes of target cells due to the presence of fusion-proteins on the membrane of the erythrocyte. In addition, the delivery system of the invention can be driven to target tissues or organs when a specific electromagnetic field is applied due to paramagnetic or super-paramagnetic nano-particles located inside the system. In particular, the nano-particles are of iron. Moreover, due to their physic characteristics, the delivery systems of the invention can function as heat source for in situ thermo-ablative therapy when a specific electromagnetic field is applied. The delivery system of the invention can be used as bioactive pro-drug carrier due to its intrinsic cell-mediated homing mechanisms. Erythro-magneto-virosomes have the capacity to phosphorylate the incorporated drugs, which remain stable until the pro-drug is driven into target cells. The different aspects of targeting that can be applied to erythro-magneto-virosomes vehicles include physical methods for directing vehicles distribution (applying appropriate magnetic and electromagnetic field at the cyclotron resonance of ions), intrinsic cell-mediated homing mechanisms (the ability to phosphorylate the incorporated pro-drugs) and the engineering flexibility of erythro-magneto-virosomes to respond to any therapeutic needs.

Chemically modified magneto nano-particles bound to coumpounds, are engineered maghemite nano-particles opportunely modified to include/link on their surface specific compounds, i.e. drugs, antibodies, small molecules, DNA or fluorochromes, driven to the site of action through external application of specific electromagnetic filed. Chemically modified magneto nano-particles bound to pro-drug are engineered nano-particles containing an active site that allows them to efficiently enter the cytoplasmic membrane.

## Summary of the invention

The invention relates generally to new cell-based drug delivery system and more specifically to "erythro-magneto-virosomes" which have the capacity to encapsulate therapeutic compounds with different biological and chemical characteristics (such as antiretroviral compounds, small molecules, phosphorylated pro-drugs, chemiotherapics), and to specifically drive the pro-drug at the target tissues or organs though application of specific electromagnetic fields at cyclotron resonance of iron ions.

It is therefore an object of the invention a compound delivery system to a target cell comprising:

- an haemoglobin-deprived erythrocyte,
- a magnetic or super-paramagnetic nano-particle located inside the erythrocyte and
- a protein located on the membrane of the erythrocyte able to induce the fusion of the membrane of said erythrocyte to the membrane of the target cell **wherein the protein is a virosome-derived protein.**

Preferably, the magnetic or super-paramagnetic nano-particle is of $\gamma Fe_2O_3$. Still preferably, the magnetic or super-paramagnetic nano-particle has a diameter between 5 to 100 nm. Yet preferably, the magnetic or super-paramagnetic nano-particle is covered by a surfactant. More preferably, the magnetic or super-paramagnetic nano-particle is further modified to comprise an additional agent.

The additional agent is preferably selected from the group of an amino or carboxy or esterified group covalently bound to a fluorescent or no-fluorescent functional group.

Preferably, the virosome is from an influenza virus virosome or a vesicular stomatitis virus virosome or an Epstein Barr virus virosome.

In another particular embodiment, the system of the invention is loaded with a pharmacological or biological therapeutic compound.

Preferably, the pharmacological or biological therapeutic compound is selected from the group of: antiretroviral compound, small molecule, pro-drug, phosphorylated pro-drug or a chemiotherapeutic compound.

It is a further object of the invention the system as described above for use as a medicament. Preferably it is being administered via aerosol, parenteral or systemic route. It is a further object of the invention a method for the preparation of the system of the invention comprising:

a) depriving isolated erythrocyte of haemoglobin;
b) incubating under appropriated conditions the haemoglobin-deprived erythrocyte with a protein able to induce the fusion of the membrane of said erythrocyte to the membrane of a target cell **wherein the protein is a virosome-derived protein,** thus obtaining an erythrocyte-protein complex;
c) purifying said complex;
d) incubating the purified complex with magnetic or super-paramagnetic nano-particles under appropriated conditions.

It is a further object of the invention a pharmaceutical composition comprising the system of the invention and pharmaceutically acceptable diluents, carrier and/or excipients.

The invention will be now illustrated by means of non limiting examples referring to the following figures.

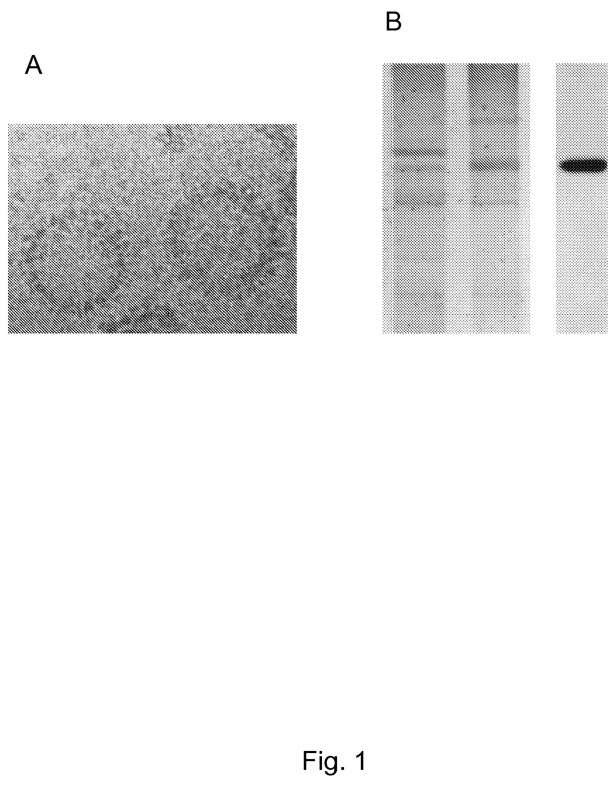

**FIGURE 1.** A: Ultra-micrograph of influenza virus; B: gel electrophoresis of HA purification from influenza virus.

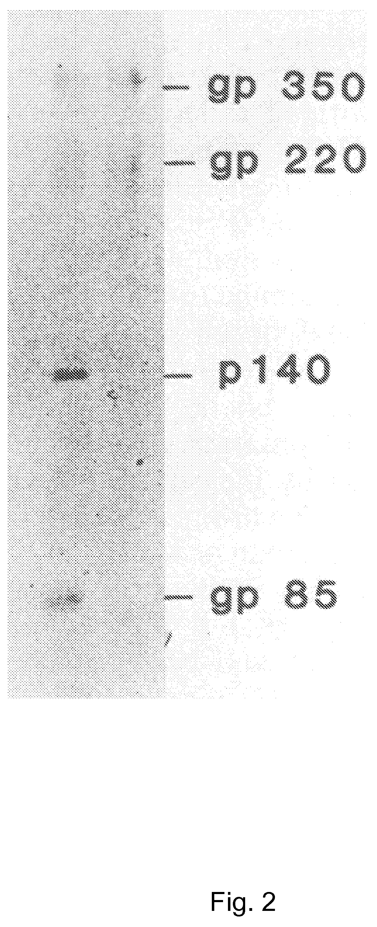

**FIGURE 2.** Gel electrophoresis of EBV(Epstein barr virus) viral spike glycoprotein purification.

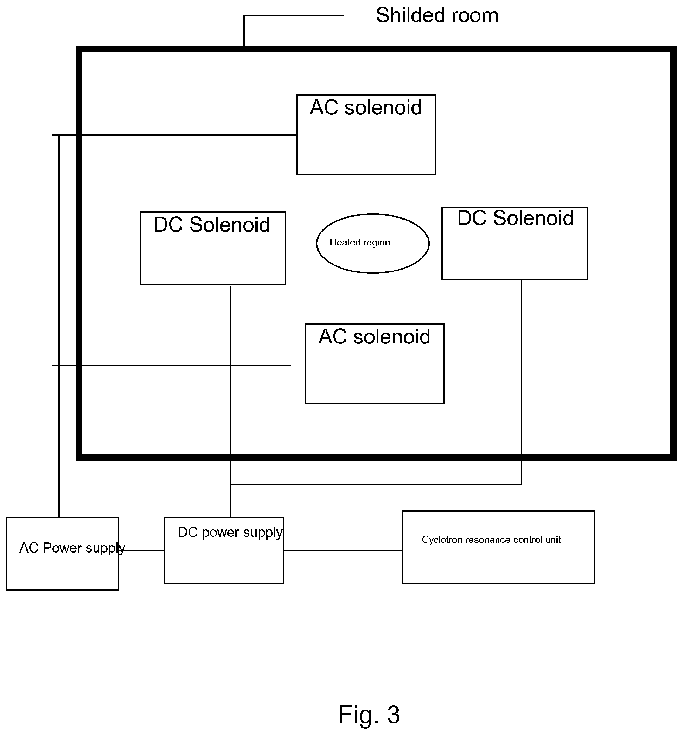

**FIGURE 3.** Schematic representation of drug delivery system driving device.

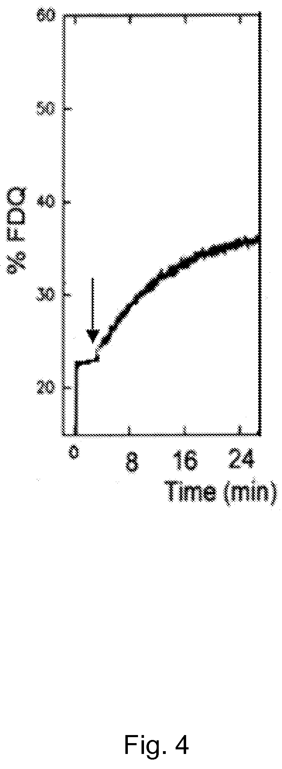

**FIGURE 4.** Octadecyl Rhodamine (R18) labeling of Erythro-magneto-HA 5-Aza-2-dC loaded and fusion with HeLa cell. Fusion has been reported as % of R18 fluorescence dequenching (FDQ) using 560 and 590 nm as excitation and emission wavelengths, respectively. Arrow indicates Erythro-magneto-HA 5-Aza-2-dC-loaded added to Hela cells.

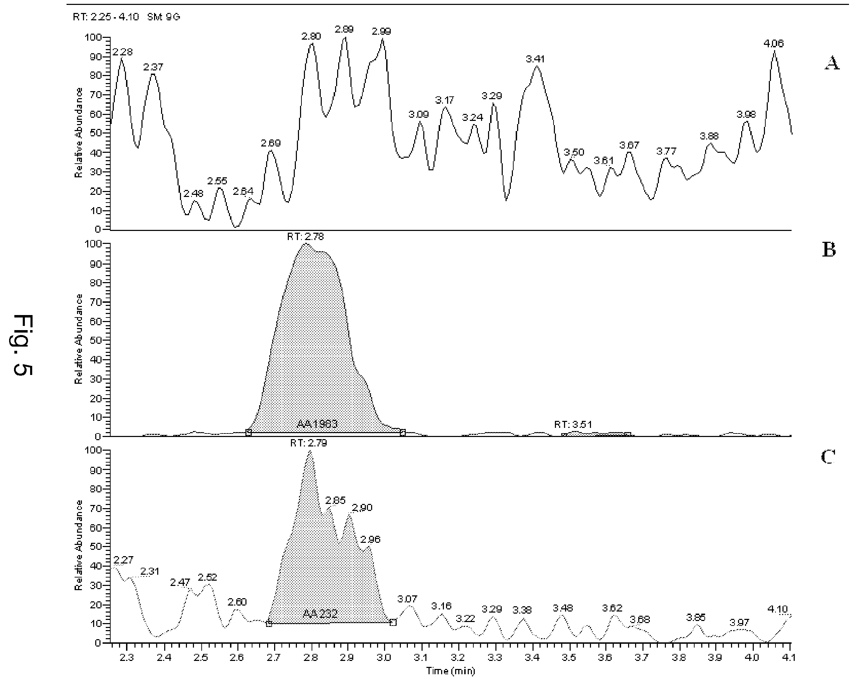

**FIGURE 5.** LC-MS/MS extracted ion chromatogram of **A**) Blank using transition 229 > 113; **B)** 5-Aza-2-deoxicytidine (decitabine, standard) at 5 $\mu$M **C)** 5-Aza-2-deoxicytidine extracted from $1 \times 10^6$ erythro-magneto-HA virosomes. The peak area ratio of sample/5-Aza-2-dC was used for quantitative analysis. The amount of 5-Aza-2-dC encapsulated into $1 \times 10^6$ erythro-magneto-HA virosomes was 0,5$\mu$M. In the figure RT= Retention Time value; AA= area value

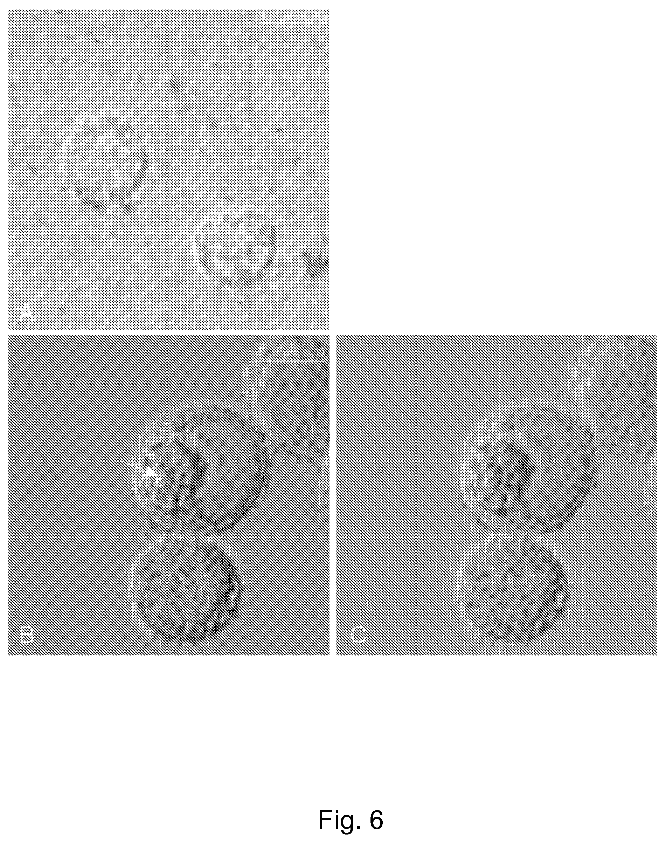

**FIGURE 6.** Confocal Laser Scanning Microscopy (CLSM) images of **A**) erytro-magneto-HA virosomes encapsulating Fluorescence-labelled superparamagnetic nanoparticles (green) and 5-Aza-2-dC; **B)** erytro-magneto-HA virosomes encapsulating Fluorescence-labelled superparamagnetic nanoparticles and 5-Aza-2-dC (arrow) fused with a HeLa cell highlighted by DAPI (4',6-diamidino-2-phenylindole staining, blue); **C)** Brightfield image of B.

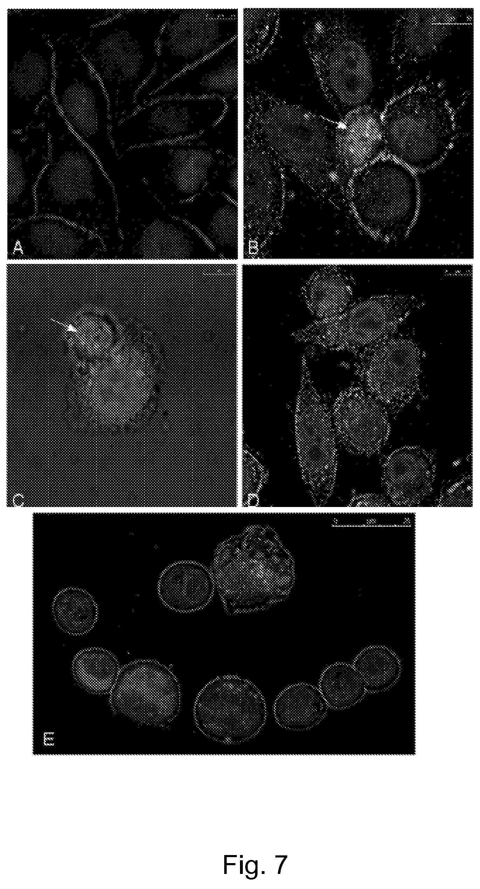

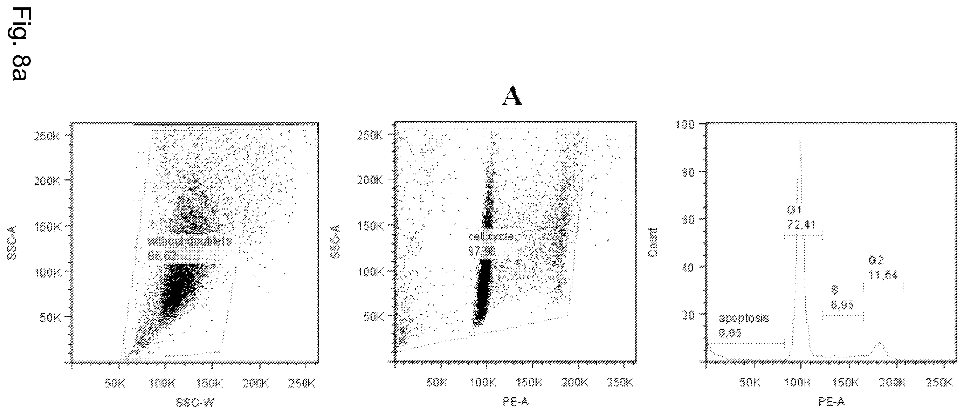

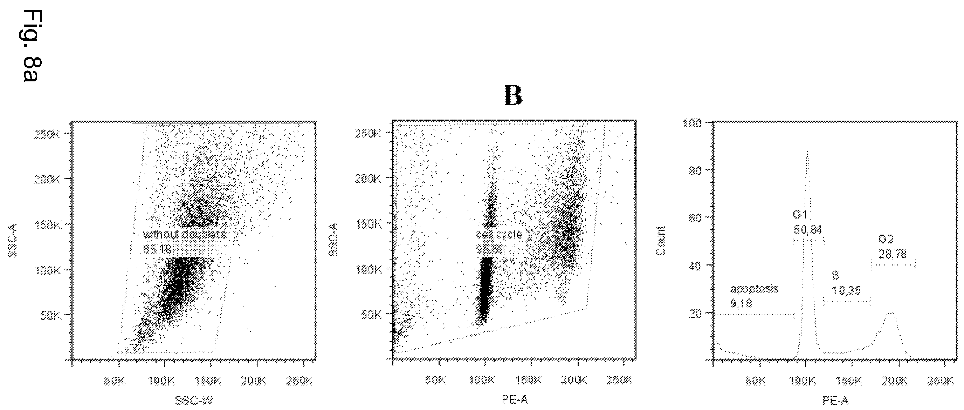

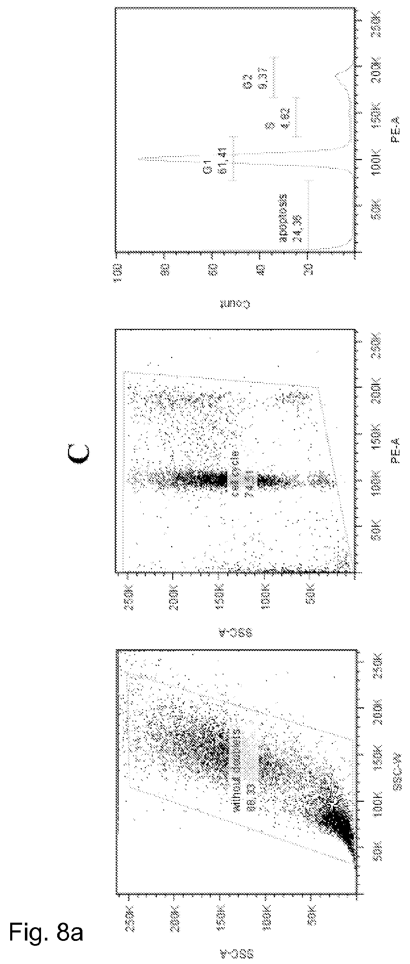

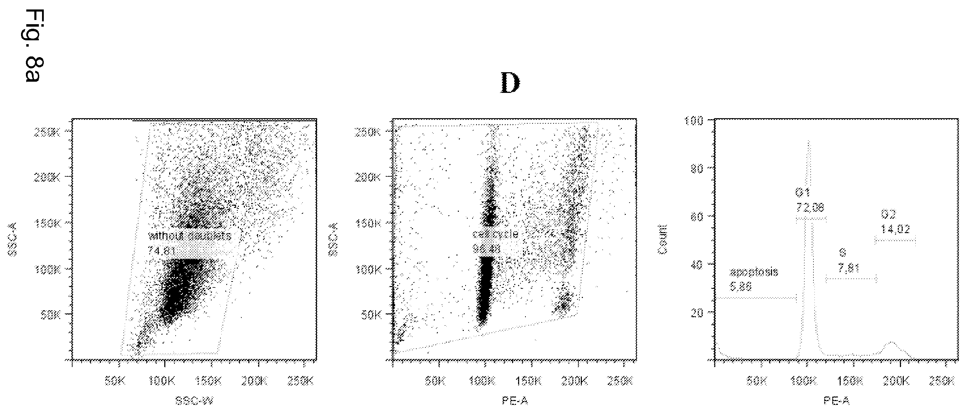

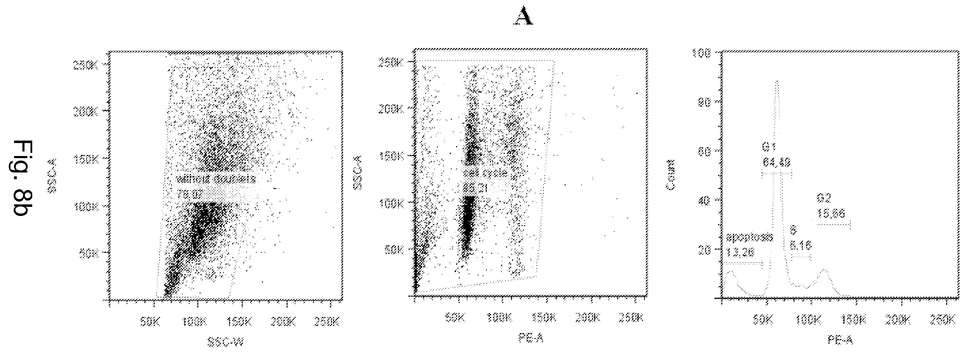

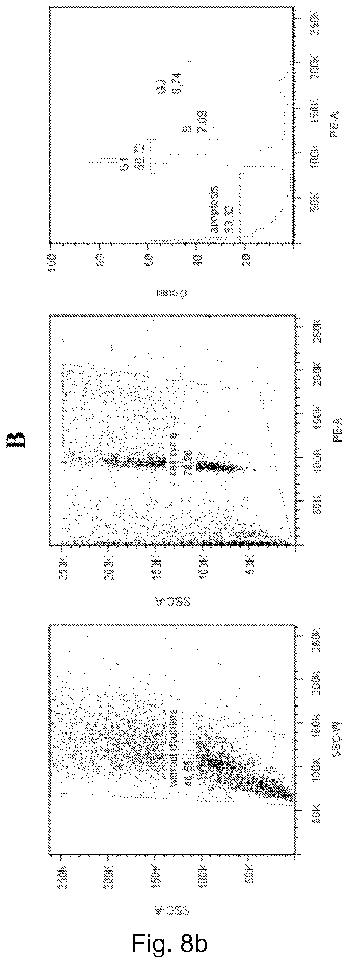

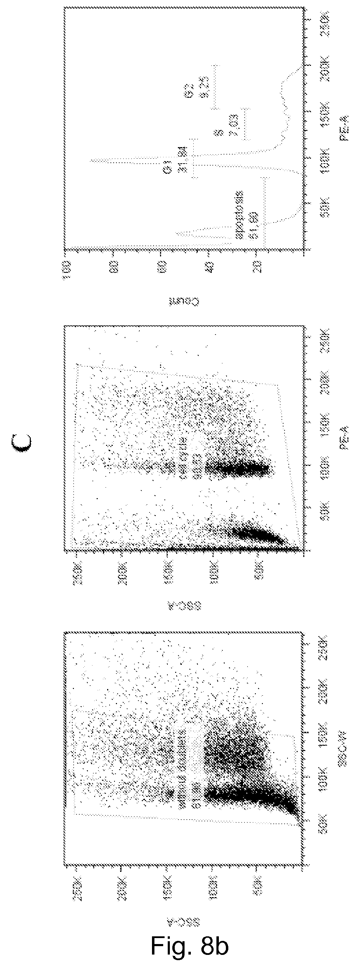

**FIGURE 7.** Confocal laser scanning microscopy (CLSM) images of HeLa cells. Timing of HeLa cells treatment with erytro-magneto-HA virosomes containing 5-Aza-2-deoxycytidine: **A)** control; **B)** 1 h after treatment; **C)** 6 h after treatment **D)** 24 h after treatment and **E)** 96 hours after treatment. HeLa cell nuclei are highlighted by DAPI staining (blue), whereas fluorescent superparamegnetic nanoparticles encapsulated in erythro-Ha virosomes are represented in green. After 1 and 6 hours of treatments (figures B and C) the erythro-magneto-HA virosome (arrows) start to fuse with cytoplasmatic membranes of HeLa cells. After 24 hours (figure D) the erythromagneto-HA virosome is completely fused with cytoplasmatic membrane of HeLa cells and the fluorescent-labeled supeparamagnetic nanoparticles and 5-Aza-2-dC are diffused inside the cytoplasm of the HeLa cells. After 96 hours of treatments (figure E) more than 45% of cells are apoptotic. **FIGURE 8a.** Propidium iodide cell cycle FACS analysis of untreated and 5-Aza-2-dC-treated HeLa cells after 48-hours - dot plots (first one without doublets and the second one with only cell cycle) and related graphics of cell cycle phases are shown. For the analysis, the Dean-Jett-Fox alogorithm has been used. **A**) control-1; **B**) treated with 2.5 $\mu$M of 5-Aza-2-dC; **C**) treated with erythro-magneto-HA-virosomes containing 5-Aza-2-dC; **D**) cell treated with supernatant in which erythro-magneto-HA virosomes containing 5-Aza-2-dC are resuspended (control-2).

**FIGURE 8b.** Propidium iodide cell cycle FACS analysis of untreated and treated HeLa cells after 96-hours - dot plots (first one without doublets and the second one with only cell cycle) and related graphics of cell cycle phases are shown. For the analysis, the Dean-Jett-Fox alogorithm has been used. **A**) control; **B**) treated with 2.5 $\mu$M of 5-Aza-2-dC; C) cells treated with erythro-magneto-HA-virosomes containing 5-Aza-2-dC.

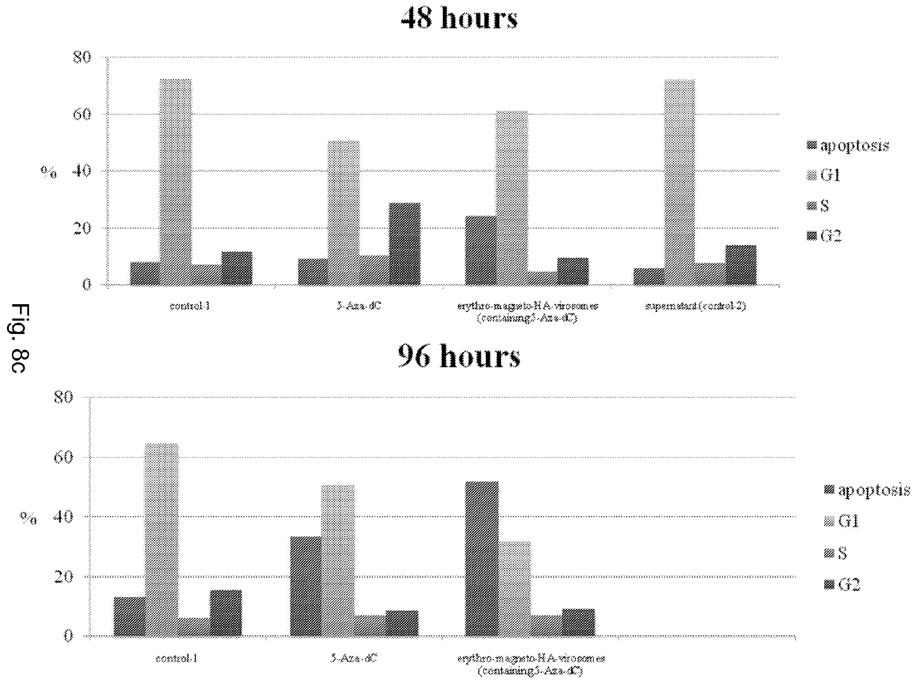

**FIGURE 8c.** Histograms showing the % of cell cycle phases (Apoptosis, G1, S and G2) of HeLa cells after (A) 48- and (B) 96-hours of treatment.

## Detailed description of the invention

VIROSOMES PREPARATION

## Influenza virus Haemagglutinin (HA) preparation and purification

### Method 1

Influenza virus obtained from the allantoic liquid of 10-days-old embryonated eggs is pelleted by ultracentrifugation and the pellet is resuspended in octa(ethyleneglycol)-*n*-dodecyl monoether ($C_{12}E_8$) and left overnight to allow for complete solubilisation of the viral membrane. The suspension is carefully homogenised and ultracentrifugated to pellet down the viral nucleocapsids. Next, the virosome suspension (supernatant containing the HA protein) is purified by ultracentrifugation on a discontinuous sucrose-gradient (50% and 10% of sucrose). The virosomes concentrate at the interface of the sucrose layers. After removal of this layer, the virosomes can be dialysed against buffer and sterilized by filtration.

### Method 2

Madin-Darby canine kidney (MDCK) cells (ATCC CC1 34) were grown in Dulbecco's modified minimum essential medium with 10% fetal calf serum. Influenza virus was prepared from the supernatant of infected Madin-Darby canine kidney cells (Davison and Sanford, 1981) at 12 h post-infection. The virus was purified by differential centrifugation and sucrose density gradient centrifugation

HA Influenza virus grown in Madin-Darby canine kidney cells contains HA in the form of its precursor, HA0. To cleave HA0, 5 $\mu$l of trypsin (10 mg/ml) was added to 1 ml of PIPES (Piperazine-1,4-bis(2-ethanesulfonic acid) buffer (5 mM PIPES, 145 mM NaCl, pH 7.5) containing 12 mg of total viral protein.

The mixture was incubated for 30 min at 37°C. The reaction was stopped by addition of 10 $\mu$l of soybean trypsin inhibitor (10 mg/ml), 2 $\mu$l of benzamidine (62 mg/ml), and 1 $\mu$l of aprotinin (1 mg/ml) in PIPES buffer. The virus was then pelleted by centrifugation (55,000 x g for 30 min) and resuspended in PIPES buffer. HAO was purified from trypsin-treated virus and non-treated virus, respectively, by solubilisation with Triton X-100 and sucrose density gradient centrifugation, as described previously. Neuraminidase was not removed by this procedure but remained at a reduced level.

The isolated HA (1 mg in 1 ml of PIPES buffer) was resolubilized by addition of 40 $\mu$l of 20% (wt/wt) Triton X-100 and by incubation for 1 h at room temperature.

Triton X-100 treatment at 4°C is often used for studies of lipid rafts to obtain detergent-insoluble glycolipid-enriched complexes. The present procedure at room temperature (25 °C) was distinct from that at 4°C in that HA trimers were not aggregated after Triton X-100 treatment as examined by gel-filtration chromatography (Sephacryl S-300, Amersham Biosciences, Uppsala, Sweden; data not shown).

## Reconstitution of HA vesicles and kinetic of fusion analysis

A lipid mixture mixed with octadecyl rhodamin (R18) (5:1.0, by weight) in chloroform/methanol (2:1) was dried and kept under vacuum overnight. The lipid film was suspended in 0.31 ml of PIPES buffer and mixed with 0.19 ml of 20% Triton X-100.

HA fusion proteins were added to the lipid solution at 1.4 mg of HA per 1 mg of lipid. To remove the detergent, the mixtures were dialyzed through Spectrapor membrane tubing 2 (25 mm, Spectrum Laboratories, Rancho Dominguez, CA) against 2.0 liters of PIPES buffer containing CaCl2 and MgCl2 at 2 mM each and 3 g of Bio-beads SM-2 (Bio-Rad) at room temperature for 4 h and then at 4°C for 80 h. The HA-reconstituted vesicles were purified by sucrose density gradient centrifugation.

Total lipid in HA vesicles was quantified by the amount of octadecylrhodamin (R18) in the vesicles, based on the fact that R18 accounts for 15% of the total weight of lipids in the vesicles. The vesicles were solubilized by adding 0.1% (final concentration) Triton X-100 in PBS (phosphate buffer saline), and the fluorescence of R18 (excitation at 560 nm and emission at 590 nm) was measured using an aliquot of the solution with a spectrofluorometer (RF5300-PC, Shimadzu, Kyoto, Japan), calibrated with R18 standard solutions containing 0.1% Triton X-100. The degree of R18 self-quenching in each HA vesicle was examined by comparison of R18 fluorescence before and after solubilization of the vesicle with 0.1% Triton X-100 in PBS.

## Vesicular Stomatitis Virus (VSV), VSV virosomes preparation and VSV G protein purification

For the determination of the critical micelle concentration (CMC) of octal glucoside (OG) different saline buffers ([HEPES] = 10 mM and 0 < [NaCl] < 1 M, pH 7.4) containing 1 $\mu$M ANS (aniline naphthalene sulphonyl chloride) were prepared. The ANS fluorescence (Aex = 380 nm, Aem = 490 nm) was recorded on a spectrofluorimeter SPEX (FILIII) connected to a computer. The buffer containing the ANS was poured directly into a quartz cuvette, which was placed in the spectrophotometer, maintained under gentle and continuous stirring, and thermo-stated at 25°C ($\pm$ 0.2°C). The evolution of ANS fluorescence was continuously monitored during the addition of a concentrated OG solution (200 mM). The rate of OG addition was controlled by a syringe pump (Perfusor VI; Braun). The OG concentration in the cuvette was then calculated over the time using the following equation (equation 1):

$$\text{Eq 1: } [OG]\text{tot} = ([OG]s * rs * t)/(v. (vo) + (rs * t)) \text{ (mM)}$$

in which [OG]s is the OG concentration in the syringe in mM, [OG]tot is the OG concentration raised in the cuvette at any time in the experiment, rs is the injection rate of the syringe pump in ml/min, t is the time in min, and VO is the initial volume in the cuvette in ml.

## Solubilization of VSV

The solubilization of intact viruses has been followed during the continuous addition of OG by measuring optical density (OD) at 550 nm on a Perkin-Elmer (Lambda 2) double-beam spectrophotometer. A volume of 1.4 ml of initial VSV solution was placed in a 1-cm optical quartz cell thermostated at 25°C ($\pm$0.2°C) and equipped with a paddle stiffer. A concentrated OG solution made in the same buffer as the one used to dilute VSV (i.e., 1 M NaCl, 10 mM HEPES, 1 mM EDTA (ethylene diammino tetraacetic acid), pH 7.4) was progressively added through thin tubing connected to a glass precision syringe pump (Perfusor VI; Braun). The OD was recorded as a function of time; this was correlated to the OG and VSV concentrations in the cuvette by Eqs. 1 (see above) and 2, respectively:

$$\text{Eq 2: } [VSV[V]stv]0to,t = 1 + ([-O\_G[tVoStV/](0O(GM])s - [OG]tot) 2$$

in which [VSV]0 is the initial VSV concentration in the cuvette, which can be expressed in mg total protein/ml; [VSV],0, is the VSV concentration in the cuvette at any time in the experiment.

## Removal of the nucleocapsid

The solubilized VSV was centrifuged for 1 h at 64,000 x g to spin down the nucleocapsid. The supenatant containing the solubilized proteins and lipids was collected.

## Reconstitution of VSV envelope by dilution

The reconstitution profiles were obtained by following the turbidity (OD at 550 nm) at 25°C (+ 0.2°C) during the continuous addition of free OG buffer to a cuvette containing 1 ml of the solubilized envelope. A volume of 2.5 ml of buffer was added to the cuvette over 3 h, giving a final dilution factor of 3.5. The OG and VSV concentrations were calculated from the time using Eqs. 3 and 4:

$$\text{Eq. 3} = [OG]t.t = [OG]o * (vo)/(vo + (r, * t)) \, (mM)$$

$$\text{Eq. 4} = LrVSVIItot \, --[V(SvVo)] = + (+ (rs * tt) \, (mg^*ovfop)rotein/ml)$$

in which [OG]o and [VSV]0 are the initial OG and VSV concentrations in the cuvette in mM and mg/ml, respectively; [OG]tot and [VSV],0, are the OG and VSV concentrations raised in the cuvette at any time in the experiment; rs is the injection rate of the buffer in ml/min; t is the time in min; and vo is the initial volume in the cuvette in ml.

**Reconstitution of VSV envelope by absorption of OG on BB SM2**

For functional reconstitution, Bio Beads SM2 were used to eliminate OG. Ten vials were prepared, each containing 20 mg wet Bio Beads SM2 for each millilitre of sample. The sample was added to the first vial and kept under gentle magnetic stirring for 12 min at room temperature (25°C). Thereafter, the sample was taken from the vial and poured into the following vial. The total duration of the elimination is about 2 h. When needed, R18 was added to the solubilized sample at a probe-to-lipid ratio of 1% before detergent removal by BB SM2 (see Fig. 1 for Ultra-micrograph of influenza virus and gel electrophoresis of HA purification from influenza virus).

**Epstein Barr Virus (EBV) and EBV Virosomes preparation**

Virus-producing P3HR1-C113 cells (ATCC, HTB62) were induced with 30 ng of 12-o-tetradecanoylphorbol-13-acetate per ml, and after 7 days virus was collected from the spent culture medium. The medium was centrifuged at 4,000 x g for 10 min to remove cells, 100 $\mu$g of bacitracin per ml was added to the clarified supernatant, and the virus was pelleted by centrifugation at 20,000 x g for 90 min. Pellets were suspended in 1/250 of the original volume of medium containing 100 $\mu$g of bacitracin per ml, reclarified by centrifugation three or four times at 400 x g, and filtered through a 1.2-,$\mu$m-pore-size filter (Acrodisc; Gelman Sciences, Inc., Ann Arbor, Mich.) and stored at -700C.

Virus proteins were extrinsically labeled with $^{125}$I (Amersham Corp., Arlington Heights, Ill.). Virus was pelleted from concentrated culture supernatant by centrifugation at 100,000 x g for 1 h, suspended in 400 ul of phosphatebuffered saline, pH 7.4, and labeled with 0.5 mCi of $^{125}$I by using Iodogen (tetrachlorodiphenylglycouril; Pierce Chemical Co., Rockford, Ill.) (10). Free iodide was removed by gel filtration on Bio-Gel P-6DG (Bio-Rad Laboratories, Richmond, Calf). Labelled virus was then repelleted before solubilisation.

*Preparation of EBV virosomes*

Virosomes were made as following described. Briefly, pelleted virions were extracted with lysing buffer containing 1% Triton X-114, 10 mM Tris, pH 7.4, 150 mM NaCl, 3 mM NaN3, 1 mM phenylmethylsulfonylfluoride (PMSF), and 100 U7ml of aprotinin, sonicated for 1 min, and then centrifuged at 100,000 x g for 1 h. The supernatant was assayed for protein concentration by using BCA protein assay reagents (Pierce) and bovine serum albumin as a standard. Extracted viral protein at a 1:5 (wt/wt) protein: lipid ratio was added to a dried mixture of L-a-lecithin (egg) and cholesterol (Avanti Inc., Pelham, Ala.) (molar ratio, 1.7:1). The detergent:lipid molar ratio was adjusted to a minimum of 6:1, and virosomes were formed by extensive dialysis against buffer (10 mM Tris, pH 8.0, 150 mM NaCl, 3 mM NaN3, and 1 mM PMSF) containing Amberlite XAD-2 (Sigma Chemical Co., St. Louis, Mo.) to remove detergent to below its critical miceilar concentration; the final dialysis was done against buffer without PMSF or NaN3. The dialysate was frozen and thawed three times and sieved over Sepharose 2B-300 (Sigma). Virosomes were collected in the void volume, pelleted by centrifugation at 16,000 x g for 30 min, suspended in buffer, and stored at -70°C.

**EBV glycoprotein gp 85 and 350/220 purification**

*DEAE chromatography.*

DEAE Sephacel (Pharmacia Fine Chemicals, Sweden) was washed with DEAE buffer (10 mM Tris, 0.1 mM EDTAL3 0.5% Triton, pH 7.8) and then poured into a Pharmacia 26/40 column to produce a bed volume of approximately 100 ml. Approximately 130 mg of the soluble extract prepared from TPA-activated 8-95-8 cells were then poured onto the column and then eluted with a 0 to 0.5 M linear NaCl gradient in DEAE buffer. Six-milliliter fractions were collected, and every fifth sample was read for conductivity with a Beckman conductivity bridge, model RC16B2 (Irvine. CA). All fractions with conductivities between 10,000 and 13,500 ohms were then pooled. Protein determinations on every other tube from the DEAE column were determined with Coomassie Blue technique, with bovine serum albumin as the standard protein.

*Ricin chromatography.*

A 5-ml column was formed with Ricinus communis agglutinin II (Ricin) bound to agarose beads (E-Y Laboratories, San Mateo. CA). The pooled extract from DEAE column was then passed through the column, and the column was washed with 30 ml of PBS plus 0.5% Triton. Glycoproteins were then eluted from the column with 0.05 M lactose and 0.05 M galactose in PBS plus 0.5% Triton. Two-milliliter fractions were collected, and each fraction analyzed for protein as described above. Positive fractions were then pooled and analyzed for the presence of gp 85 and gp350/220 (see Fig. 2 for gel electrophoresis of EBV viral spike glycoproteind purification).

## ENGINEERING ERYTHROCYTES WITH FUSION PROTEINS

An aliquot (2 ml) of packed, freshly collected human blood are incubated with fusion proteins (HA influenza viral spike glycoprotein or VSV G viral spike glycoprotein or EBV viral spike glycoprotein) prepared as described above at 37 °C for one hour, the fusion efficiency followed spectrophotometrically by the octadecyl rhodamin (R18) fluorescence test.

## Method 1

## Erythro-virosomes (ghosts) preparation and incorporation of super paramagnetic nano-particles and drugs

Human Red Blood Cells (RBCs) are obtained from blood of healthy donors by centrifugation at 1700 rpm for 10 min at 4°C. Freshly collected RBCs are washed twice in physiological phosphate-buffer saline (1X PBS) (137mM NaCl, 5.7 mM KCl, 5,4 mM $Na_2HPO_4$, 4,5 mM $KH_2PO_4$ pH 7,4). The RBCs are than lysed in lysis buffer (10mM TRIS 0.1 mM EDTA, 1mM $MgCl_2$ at pH 7.2) for 60 minutes at 0°C.

The isotonicity of RBCs deprived of haemoglobin is restored adding 1X resealing buffer (137mM NaCl, 5.7 mM KCl, 5,4 mM $Na_2HPO_4$, 4,5 mM $KH_2PO_4$, 15mM $MgCl_2$ pH 7,4) supplemented with appropriate doses of therapeutic compound, superparamagnetic nanoparticles (magnetite-maghemite) and of viral spike glycoprotein (HA or VSV or EBV) at concentration spanning from $25\mu g$ to 1,4 mg of viral spike glycoprotein per 1 mg of lipids. In particular, the isotonicity of $5 \times 10^7$ haemoglobin-deprived RBCs was restored adding 10ml of 1X resealing buffer (137mM NaCl, 5.7 mM KCl, 5,4 mM $Na_2HPO_4$, 4,5 mM $KH_2P0_4$, 15mM $MgCl_2$ pH 7,4) supplemented with 2,5 mM 5-Aza-2-dC (decitabine, Sigma-Aldrich ,St Louis, MO, USA), 25 mg of 100nm superparamagnetic nanoparticles (magnetite-maghemite) green-labelled (nano-screenMAG, Chemicell Company) and $50\mu g$ of HA influenza viral spike glycoprotein. The suspension was incubated for 45 minutes at 37°C under mild agitation. Sealed haemoglobin-deprived RBC, containing HA fusion protein on lipid membrane and both 5-Aza-2-dC and super-paramagnetic nano-particles inside, were collected by centrifugation at 10,000 rpm for 15 minutes in at 4 °C. The sealed haemoglobin-deprived RBCs were washed twice with 10 ml of 1X PBS by centrifugation at 9,000 rpm for 15 minutes in at 4 °C and re-suspended in 1X PBS containing 1mM $MgCl_2$ and 1% FCS.

The purified erythro-virosomes can be also lysed by rapid dilution in 300 ml of ice-cold lysis buffer (5 mM EDTA, 5 mM HEPES, pH 7.4). The lysed red blood cells (RBC) are then pelleted by centrifugation at 27 000$\times g$ for 40 min at 4°C. The pellet is re-suspended in lysis buffer and pelleted again three times or until the RBC membrane preparation became white. The resulting lysed RBC virosomes (haemoglobin-deprived RBCs) are resealed in the presence of both the magnetic or super para magnetic nano-particles (magnetite-maghemite) and the appropriate therapeutic doses of compounds (i.e chemical drugs, antibodies, DNA, small molecules, fluorochromes) at 37°C for 1 hour.

The erythro-magneto virosomes are then isolated again by centrifugation at 27 000$\times g$, suspended in buffer and stored in small aliquots at -20°C until required.

Total lipids in erythro-magneto-virosomes are quantified by the amount of octadecyl rhodamine (R18) in the haemoglobin-deprived RBCs, based on the fact that R18 accounts for 15% of the total weight of lipids in the haemoglobin-deprived RBCs. The erythro-virosomes are solubilized by adding 0.1% (final concentration) Triton X-100 in PBS, and the fluorescence of R18 (excitation at 560 nm and emission at 590 nm) is measured using an aliquot of the solution with a spectrofluorometer, calibrated with R18 standard solutions containing 0.1% Triton X-100. The degree of R18 self-quenching in each erythro-virosomes is examined by comparison of R18 fluorescence before and after solubilization of the erythro-virosomes with 0.1% Triton X-100 in PBS.

**Method 2**

**Engineering erythrocytes with fusion proteins**

Human RBCs are obtained from blood of healthy donors by centrifugation at 1700 rpm for 10 min at 4°C. Fusion protein (HA or VSV or EBV viral spike glycoprotein) is added to the solution where the haemoglobin-deprived RBCs are re-suspended at concentration spanning from 25μg to 1,4 mg of viral spike glycoprotein per 1 mg of lipids. To remove the detergents, the mixtures are dialyzed through Spectrapor membrane tubing 2 (25 mm, Spectrum Laboratories, Rancho Dominguez, CA) against 2.0 liters of PIPES buffer containing CaCl2 and MgCl2 at 2 mM each and 3 g of Bio-beads SM-2 (Bio-Rad) at room temperature for 4 h and then at 4 °C for 80 h. The virosomes-reconstituted vesicles are purified by sucrose density gradient centrifugation.
The Red Blood Cells (RBC) virosomes complex are purified by centrifugation to remove non fusogenic viral fusion proteins.

**Transient lysis of engineering erythrocytes with fusion proteins and incorporation of super paramagnetic nano-particles and drugs**

RBCs virosomes complex are washed twice in HEPES Buffer (10mM Hepes, 140mM NaCl, 5mM glucose at pH 7,4) and then resuspended at 70% hematocrit in the same solution. RBC-virosomes are dialyzed for 20 min using a tube with a cut-off of 12 kDa against 10 vol. of lysis buffer (10 NaHCO$_3$, 10 mM NaH$_2$PO$_4$, 20 mM glucose, 4 mM MgCl$_2$, at pH 7.4) containing therapeutic compounds and super paramagnetic nano-particles (transient lysis). After incubation at 37°C for 10 min, resealing of dialysed ghosts is performed by adding 0.1 vol. of resealing buffer (5 mM adenine, 100 mM inosine, 2mM ATP, 100 mM sodium pyruvate, 100 mM glucose, 194mM NaCl, 4mM MgCl2, 1,606 M KCl at pH 7.4) and further incubation at 37°C for 30 min. Finally, erythro-magneto-virosomes, which have encapsulated the therapeutic compound, are washed four times in Hepes buffer and re-suspended in the some buffer at 45% hematocrit.
The erythro-magneto-virosomes containing the desired therapeutic compound are driven to the desired specific target site for instance by applying an external static magnetic field. The fusion of erythro-magneto-virosomes with target cells may be triggered by an appropriate external resonating electromagnetic field generated by a specific apparatus for culturing eukaryotic cells as for instance the one described in the patent application WO/2007/004073.

MAGNETO-NANO PARTICLES

***Binding of magneto nano-particles with therapeutic compounds***

Paramagnetic or super-paramagnetic nano-particles can be chemically modified with active group enabling them to be efficiently transported trough the plasma membrane, thus, carrying the pro-drug in the active form. Magnetic nano-particles of iron maghemite ($\gamma$Fe$_2$O$_3$) may be used. The particles have a diameter spanning from 5 to 100 nm (5, 10, 20, 30, 40, 50, 60, 70, 80, 90, 100 nm) and may be covered with surfactants such as Pluronic, dichlorobenzene, oleic acid, triphenylphosphine, oleyl amine, trioctylphosphine oxide, KorantinSH, dodecyltrimethylammonium bromide, didodecyldimethylammonium bromide to inhibit their aggregation. These iron maghemite nanoparticles can be further opportunely modified to include/link on their surface specific active groups such as amino or carboxy groups covalently bound to fluorescent functional group and/or other compounds. The active groups in their acetyl, esterified, methylated or acethyl-methyl-esterified forms can bound any compounds. When nanoparticles are conjugated with target agents or undergo any surface modification, particle agglomeration as a result of their large surface-to-volume ratio becomes a primary concern to avoided. When nanoparticles agglomerate, they not only lose their intended functionality, but can also be quickly cleared by macrophages or accumulated in the reticule-endothelial system before they can reach the target cells. One approach to solving this problem is to modify the particle surface with poly(ethylene glycol) (PEG) self-assembled monolayers. Surfaces covered with PEG have proven to be non-immunogenic, non-antigenic, and protein resistant. While the PEG moiety provides an efficient system to increase particle circulation time in blood protecting them from aggregation and from fast clearance, the nano-particle systems may also be coupled with tumor targeting agents to be useful for the intended applications. Thus, the PEG moiety immobilized on nano-particles may also provide an active functional group capable of conjugating with targeting agents. PEG is used widely to functionalize proteins and peptides for drug delivery. Research in cell targeting has also utilized functional PEG molecules conjugated with folic acid on liposomes. Monofunctional PEG molecules coupled to proteins are known to prolong the particle circulation time in blood, and reduce immunogenicity. While functionalized carboxyl or amine PEGs are widely available, they remain expensive and require chemical modification to convert to their corresponding silanes. Further, these functional PEGs are available mainly in high molecular weights, which may inhibit PEG monolayer self-assembly on the nanoparticle surface due to the labile nature of PEG molecules. Currently available PEGs can conjugate with only one type of functional group present in targeting agents, typically either amine or carboxyl groups, and, in most of cases, they are not suitable for nanoscaled devices such as nanoparticle systems due to their high molecule weight. PEG silylation normally occur in organic solution, whereas conjugation with tumor targeting agents such as folic acid or antibodies needs to be conducted in aqueous solution. Thus, PEG self-assembled monolayers (SAM) must be flexible enough to prevent agglomeration during solvent exchange and remain active in solvents to provide a terminus for conjugation. Despite the advances in the use of nanoparticles as contrast agents and drug carriers noted above, a need exists for nanoparticles that can be surface-modified to function as both contrast enhancement agents and drug carriers simultaneously, allowing real-time monitoring of tumor response to drug treatment. The present invention seeks to fulfil this need and provides further related advantages. Synthesis of magnetic materials on the nanoscale is a topic of great currently, because of interest the novel microscopic properties shown by particles of quantum dimensions located in the transition region between atoms and bulk solids. Nanoparticles have physical and chemical properties that are characteristic of neither the atom nor the bulk counterparts. Quantum size effects and the large surface area of magnetic nanoparticles dramatically change some of the magnetic properties and exhibit superparamagnetic phenomena and quantum tunnelling of magnetization, because below a critical size, nanoparticles become single domain. Because of the unique physical properties, super-paramagnetic nanoparticles have great potential for several applications in different areas such as ferrofluids, color imaging, information storage, and cell sorting. As is known, below a critical size, nanoparticles will exhibit superparamagnetism. The hysteresis loop is usually used to study the superparamagnetism of the particles. It is generally considered that once the hysteresis loop exhibits a curve, we can say that the particle has superparamagnetism.

***Synthesis of Nanoparticles***

Ferric chloride hexahydrate ($FeCl_3 + 6H_2O > 99\%$) and ferrous choride tetrahydrate ($FeCl_2 + 4H_2O > 99\%$) are used as iron sources, and aqueous ammonia is used as the precipitator. Distilled water is used as the solvent. The solution of $FeCl_3$ and $FeCl_2$ is mixed with certain molar ratio. The corresponding phase $NH_4OH$ is slowly injected into the mixture of $FeCl_3$ and $FeCl_2$ under vigorous stirring. After precipitation, the $Fe_3O_4$ particles must be repeatedly washed and filtered before drying at room temperature in air atmosphere to form powders. The chemical reaction of $Fe_3O_4$ precipitation can be described as follows:

$$Fe^{2+} + 2Fe^{3+} + 8OH^- \text{------- } Fe_3O_4 + 4H_2O$$

Before the reaction, $N_2$ gas must flown through the reaction medium to prevent possible oxidation. The reaction is operated in a closed system to provide a non oxidation environment. Otherwise, $Fe_3O_4$ might also be oxidized as follows:

$$Fe_3O_4 + 0.25O_2 + 4.5H_2O \text{------- } 3\ Fe(OH)_3$$

which means the suspension will turn from black to yellow and will affect the purity of the final production. The $Fe_3O_4$ nano-particles prepared by co-precipitation dried at room temperature in air atmosphere to form a powder, which we called the powder sample. $Fe_3O_4$ powder, 0.0122g, is dispersed into 6 ml distilled water to form the aqueous solution and then HCl added to the aqueous solution to a pH of 2. After ultrasonic shocking of the aqueous solution, the $Fe_3O_4$ nano-particles are dispersed in the distilled water. The structural properties of $Fe_3O_4$ nano-particle powders then are analyzed by X-ray diffraction (XRD; D/ MAX3B) using Cu Ka-radiation. The XRD intensity data are collected over the range 20° , 2v , 60° at room temperature. The mean size and morphologies of $Fe_3O_4$ particles observed by transmission electron microscopy.

***Magnetic nanoparticles coating and functionalization***

*Coating*

Isolated magnetic nanoparticles are than diluted in water at 5-30 mg/ml concentrations. A 5-40% aqueous solution of PEGylated ligand added to the suspension slowly during continuous sonication at 50-60°C until all or most of the monolayer-coated particles form a stable aqueous suspension when placed a top a handheld magnet. Than the PEG coated nanoparticles are acidic functionalized (---COOH) by proper reaction methods with tri-ethylamine ($Et_3N$) and dimethylaminopyridine (DMAP) as catalyst in $CH_3CN$.

*Functionalization of magneto-nanoparticles with esters groups*

Acetoxymethyl moieties are selectively linked to the nano-particles surface through carboxylic functions. Esters are obtained using acetoxymethylchloride (prepared using standard methods) with tri-ethylamine ($Et_3N$) and dimethyl-

aminopyridine (DMAP) as catalyst in $CH_3CN$.

### Linkage of drugs/compounds to Chemically modified magneto-nanoparticles

Drugs, pro-drug or any therapeutic compounds covalently linked onto the surface of nano-particles by esterification of one of the available hydroxyl functions of the furanosyl moiety of the drug on the carboxylic function on the particle surface. If necessary, a short alkyl- or alkoxyl-spacer may be introduced between the drug and the particle. The reaction is carried out using suitable carbodiimide activation. To ensure cell permeability of the drug-nanoparticle conjugate, acetoxymethyl esters are also introduced, either directly on the nanoparticle surface (according to the method developed and described before), or trough further functionalization of the drug molecule.

The second hydroxyl function of the sugar is used to introduce an acetoxymethyl group. This double functionalization is obtained by introduction of suitable semi-permanent protecting groups on the drug molecule.

### Administration of magneto-drugs and erythro-magneto-virosomes

The administration route of both magneto-drugs (maghemite nano-particles opportunely modified to include/link on their surface specific compounds, i.e. drugs, antibodies, small molecules, DNA or fluorocromes, driven to the site of action through external application of specific elelctromagnetic filed) or erythro-magneto particles (erythrocytes containing inside or on their surface maghemite nano-particles which can be realised at the target tissue or cells and induce local thermal effect if specific electromagnetic filed is applied) or erythro-magneto-virosomes. Erythro-magneto-virosomes are magneto erythrosomes containing on their surface viral fusion proteins from influenza or para influenza virus essential for increasing the efficiency of fusion of erythro-magneto-virosomes with cell target cytoplasmatic membranes to release the therapeutic compounds and/or maghemite, encapsulated inside the erythro-magneto-virosomes, into target cells for instance via aerosol, by parental or systemic injection.

Magneto-drugs, erythro-magneto-virosomes or erythro-magneto particles can be directed to the site of action through external application, via laparoscopy micro magneto plaques or by using the specific apparatus for culturing eukaryotic cells able to produce specific magnetic and electromagnetic filed at the cyclotron resonance of ions as described for instance in the patent application WO/2007/004073.

### Method for driving drug delivery system and generating hyperthermia in a field free region using superparamagnetic nanoparticles

Nano-particles may be magnetically driven to site of action, then drug may be released by applying appropriate sinusoidal and static magnetic tuned at iron cyclotron energy resonance. After drug being released it is also possible to proceed with thermal ablation. Heat therapies such as hyperthermia and thermoablation are very promising approaches in the treatment of cancer. Compared with available hyperthermia modalities, magnetic fluid hyperthermia yields better results in uniform heating of the deeply situated tumors. In this approach, fluid consisting of super-paramagnetic particles (magnetic fluid) is delivered to the tumor. An alternating (AC, alternate current) magnetic field is then used to heat the particles and the corresponding tumor, thereby ablating it. However, one of the most serious shortcomings of this technique is the unwanted heating of the healthy tissues. In our system we demonstrated that by depositing appropriate static and alternating magnetic fields, both tuned in order to achieve a particular iron ion cyclotron frequency, focused heating of the magnetic particles can be achieved.

Currently, various types of heat treatment modalities are available for the treatment of cancer such as microwave, ultrasound, focused ultrasound, radio frequency (RF) capacitance hyperthermia, RF probe hyperthermia, and magnetic fluid hyperthermia (MFH). Each treatment technique has certain limitations. For instance, microwave hyperthermia has a poor depth of penetration, which makes it unsuitable for treatment of deep-seated tumors. Compared with microwave, ultrasound has better penetration depth and focusing abilities, but particular drawbacks of ultrasound include high energy absorption of the bone and liquid-containing organs RF capacitance hyperthermia, the major limiting factor is the inability of the electric field to focus on the tumor, so that all the tissues that the electric field penetrated are heated. RF probe hyperthermia has the disadvantage of poor accessibility to the deep-seated tumors, with a limited accuracy of localization. Finally, this technique is not suitable for the treatment of large tumors.

The patent application WO/2007/004073 concerns a system that focuses the heat into very small regions so that focused heating of magnetic particles, and therefore tumors, can be achieved by limiting the damage to the collateral healthy tissue. This technique envisages increasing the permeability of a given ion through a membrane, which is subject to the 20 earth's static magnetic field or to any other static magnetic field which is arbitrarily chosen, superimposing on this static magnetic field a variable magnetic field modulated with a frequency F proportional to the ratio $Q/m$ of charge $Q$ to mass $m$ of the ion in accordance with 25 the relation which defines the cyclotron frequency $Fe$, 2rr $Fc = Q/m$ $Eo$ where Q and $m$ are the charge and the mass of the ion expressed respectively in Coulombs and in kg, and Eo is 5 the intensity of the static magnetic field expressed in Tesla. This technique, which envisages the use of Helmholtz coils for the generation of the variable magnetic field, is claimed to allow regulation, at a speed and with a degree of precision hitherto unknown, of 10 the ion exchange between the outside and the inside of a cellular body. It is well known that, under alternating magnetic fields, super-paramagnetic (single domain) nanoparticles are heated as a result of the rotation of the particle itself (Brownian relaxation) and the rotation of the magnetic moment inside the particle (Neel relaxation). If a static magnetic field with equal or larger amplitude is superimposed on the alternating field, the single domain particles and their magnetic moments will align with the static field. In this way, Neel and Brownian relaxations will be blocked and the heating of the particles will be diminished. Has been shown that a static magnetic field, perpendicular to the AC field, applied to magnetic fluids (colloidal dispersions of super-paramagnetic iron oxide particles) and it was observed that heating was significantly reduced when the amplitude of the static field approached that of the alternating field. These studies demonstrate that static magnetic fields can be used to modulate the heating effect of AC magnetic fields on the magnetic domains. In traditional MFH systems, magnetic particles that have been dispersed into the tissue are heated by application of AC magnetic fields. In this process, unselective heating of the particles occurs because all the particles exposed to the alternating field are heated equally. By depositing appropriate dc magnetic field gradients on the AC magnetic fields, one can generate AC field dominant regions and achieve focused heating of the magnetic particles in these regions. The static field vectors generated by the solenoids cancel each other at the center of the system and a region with a very small de magnetic field is formed around the center, which can be named as the field free region (FFR). If an alternating magnetic field calculated matching the iron cyclotron energy resonance in earth static magnetic field shielded condition, is applied to the space between the solenoids, the alternating field will be dominant in the FFR and only the magnetic particles inside the FFR will be heated. The particles outside this region cannot be heated as a result of the dominance of the static field on the alternating field. The field-free region explained above can be reduced further (i.e., more intense heat focus can be obtained) by increasing the current magnitudes flowing through the dc solenoids (see Fig. 3 for a schematic representation of drug delivery system driving device.

**Kinetics of Erythro-magneto-HA virosomes fusion with Hela cells: Spectrofluorimeter analysis**

Erythro-magneto-HA virosomes were prepared as described above and were labelled by adding Octadecyl Rhodamine (R18). The Erythro-magneto-HA virosomes/R18 loaded were added to Hela cells. Total lipid in erythro-magneto-HA virosomes/R18 was quantified by the amount of Octadecyl Rhodamine (R18) in their membrane, based on the fact that R18 accounts for 15% of the total weight of lipids on it. The erythro-HA-virosomes/R18 were solubilized by adding 0.1% (final concentration) Triton X-100 in PBS, and the fluorescence of R18 (excitation at 560 nm and emission at 590 nm) was measured using an aliquot of the solution with a spectrofluorometer (RF5300-PC, Shimadzu, Kyoto, Japan), calibrated with R18 standard solutions containing 0.1% Triton X-100. The degree of R18 self-quenching in each erythro-HA virosomes was examined by comparison of R18 fluorescence before and after solubilization of the erythro-HA virosomes with 0.1% Triton X-100 in PBS. Erythro-magneto-HA virosomes were added to Hela cells. The kinetic of erythro-magneto-HA virosomes fusion with Hela cells was calculated as % of R18 fluorescence dequenching (FDQ) using 560 and 590 nm as excitation and emission wavelengths respectively (see Fig. 4).

**High pressure liquid chromatography (HPLC) analysis of 5-Aza-2-dC incorporation in Erythro-magneto-HA virosomes**

*Chemicals and solutions*

5-Aza-2-deoxycitidine (Decitabine) was purchased from Sigma-Aldrich (St Louis, MO, USA). Ammonium acetate was from BDH (Poole, UK). HPLC grade acetonitrile (MeCN) and methanol was from Rathburn (Walkerburn, UK).

*HPLC analysis of Erythro-magneto-HA virosomes countaining decitabine and of decitabine standard solution*

$1 \times 10^6$ erythro-magneto-HA virosomes were incubated with lysis buffer (10 mM Tris, 0.1 mM EDTA, 1mM $MgCl_2$) to release the incorporated 5-Aza-2-dC drug and used for HPLC analysis or stored in a -80 °C freezer until analysis. Stock standard solution of decitabine at concentration of 5, 10 and 25 $\mu$M were prepared individually in methanol and stored at -80 °C conditions until analysis.

For preparation of calibration curves concentrations of 1, 5, 10 and 15m$\mu$M were used for decitabine. A mixed internal standard solution in water was freshly prepared on the day of analysis at 5 $\mu$M for decitabine. The peak area ratios of sample/5-Aza-2-dC (decitabine, standard) was used for quantitation. The limit of detection (LOD) was defined as three times the signal-to-noise ratio. The lowest limit of quantitation (LLOQ) was defined as the lowest level of analyte that could be reliably detected and reproducible with a precision of ≤20% and accuracy of 80-120% (see Fig. 5).

*Instrumentation*

The HPLC system comprised of a Dionex (Sunnyvale, CA, USA) 3000 Ultimate series LC connected to a linear ion Trap LTQ-Orbitrap (Thermo Fisher Scientific, USA) mass spectrometer, equipped with an electrospray ion source. Data were acquired and processed with Excalibur 2.1 software. Compounds were separated on a Gemini C18 (150mm-2.0mm I.D.) and 3$\mu$m particle size (Phenomenex, Torrance, CA, USA) protected by a Phenomenex Gemini C18 (4.0mm-2.0mm I.D.) and 3 $\mu$m particle size guard cartridge. The HPLC method used gradient elution; mobile phase solvent A was water with 0,1 % formic acid and mobile phase B was acetonitrile with 0,1% formic acid. The initial mobile phase composition of 92% solvent A and 8% solvent B was maintained for 2min. Between 2 and 9 min the percentage of mobile phase B was increased to 35% and then back to initial the mobile phase composition within 0,1 min, with a total time of 14 min.

The column was set at a flow rate of 0.2 ml min-1 and a temperature of 36°C. Sample volume of 15 $\mu$l was used for all LC-MS experiments. The mass spectrometer was operated in positive electrospray mode. The capillars temperature was 275 °C and the spray voltage 4.5 kV was used.

**Tumor Hela cells treatments**

Human Hela cells were purchased from the American Type Culture Collection (Rockville, MD). The cells were maintained in DMEM (Dulbecco's Modified Eagle Medium) medium supplemented with 10% fetal bovine serum, 2 mM L-glutamine, at split ratio of 1:4 twice a week.

For treatments, cells were seeded at a density of 1 x $10^6$ cells/ 3 ml of culture medium in 6-well microtiter plates. Two sets of experiments were run in parallel for both Confocal Laser Scanning Microscopy (CLSM) and FACS analysis. For CLSM analysis, on the bottom of the culture plates there were placed glass coverslips on which the cells were let grow.

After 24 hours, the culture medium was changed to media containing 2.5 $\mu$M 5-Aza-2-deoxycytidine (5-Aza-2-dC) (Sigma-Aldrich) alone or 3X$10^6$ erythro-magneto-HA virosomes containing 5-Aza-dC or buffer in which the erythro-HA-virosomes were resuspended (control-2). After 1, 6, 24 and 96 h of incubation, treated cells (with 5-Aza-dC alone or with erythro-magneto-HA virosomes containing 5-Aza-2-dC or with buffer in which the erythro-HA-virosomes where resuspended and untreated (control-1) cells were analyzed by CLSM and FACS analysis.

**Image analysis of fusion events of erythro-magneto-virosomes with tumor cells: Confocal Laser Scanning Microscopy analysis**

Tumor Cells grown on slides in presence of erythro-magneto-virosomes containing 5-Aza-2-dC were washed in PBS buffer and fixed with 4% paraformaldehyde in 1X PBS. Cell nuclei were counterstained with DAPI and mounted in antifade medium. Fluorescent and brightfield images were obtained by a Leica TCS SP5 inverted microscope system, equipped with five lasers emitting from the UV to the visible (405 Diode, Argon, HeNe 543, HeNe 594 and HeNe 633). The DAPI fluorescence was detected at excitation of 405 nm and emission of 454 nm while the green fluorescence of superparamangetic nanoparticles at excitation of 543 nm and emission of 613 nm (see Fig. 6 and 7).

**Cytofluorimetric analysis (FACS analysis)**

FACS analysis was carried out on HeLa cells treated with 2,5 $\mu$M 5-Aza-dC or 3X$10^6$ erythro-magneto-HA-virosomes containing 5-Aza-2-dC and compared to those untreated (control-1) after 48 and 96 hours of culture. FACS analysis was also carried out on HeLa cells treated with buffer in which the erythro-HA-virosomes were resuspended to check the absence of unincorporated 5-Aza-2-dC drug (control-2). Cells were fixed in ethyl alcohol and the nuclei were stained with 25 mg/ml of propidium iodide and incubated with 1 mg/ml of RNases for 1 h at 37°C. The nuclear DNA content, which discriminates the cell cycle phases, was determined using flow cytometry using Becton-Dickinson FAC-Scan ControlI (see Fig. 8a,b,c).

PRE-CLINICAL STUDIES

**Pre-clinical studies on human tumor xenografted CD1 *nu/nu* (nude) mice**

To evaluate the in vivo efficiency and efficacy of the present invention drug delivery system encapsulating therapeutic compounds and to set the best therapeutic conditions for an innovative therapies based on erythro-magneto-virosomes delivery system, CD1 *nu/nu* (nude) mice are xenografted with human tumor cells and treated with 5-Aza-dC or other anti-blastic drugs delivered by erythro-magneto-virosomes. To determine the effect of treatments, the mean tumor mass of treated mice is evaluated measuring their size with a caliper and by ultrasound. Additionally, immuno-histochemical analysis is performed on collected frozen tumor tissues of treated and control mice to evaluate the effects of treatments at histological level. Moreover, the gene expression profile on collected frozen tumor tissues of treated and control mice by using RT-qPCR analysis is analyzed to evaluate the effect of treatment at the molecular level.

**Human tumor xenografted CD1 nu/nu (nude) mice treated with erythro-virosomes delivering magneto-nanoparticles and therapeutic compounds.**

*Tumor induction in a CD1 nu/nu animal model and their treatments.*

CD1 nu/nu (nude) female mice of 6-7 weeks of age, are injected on their flank with $10^6$ human tumor cells resuspended in Matrigel. The growth of the tumor is evaluated every three days once the mass is palpable. These mice are treated with erythro-virosomes delivering magneto-nanoparticles and therapeutic compounds injected on the tail following the experimental setting shown in Table 1.

**Table 1: Tumor induction in a CD1 nu/nu animal model and their treatments.**

| CTRL Group# 1 (CD1 nu/nu) | Group #2 (CD1 nu/nu) | Group #3 (CD1 nu/nu) | Group #4 (CD1 nu/nu) |
|---|---|---|---|
| 12 mice | 12 mice | 12 mice | 12 mice |
| 2 sub groups of 6 mice each intraperitonal injection of saline solution | 2 sub groups of 6 mice each Intraperitonal injection of 5-Aza-dC | 2 sub groups of 6 mice each Tail injection of erythro-magneto-virosomes | 2 sub groups of 6 mice each Tail injection of erythro-magneto-virosomes containing 5-Aza-dC |
| 2 sacrification times (four and six weeks following treatment) | 2 sacrification times (four and six weeks following treatment) | 2 sacrification times (four and six weeks following treatment) | 2 sacrification times (four and six weeks following treatment) |

*Example of therapy with 5-Aza-dC as active anti-blastic drug*

The 5-Aza-dC treatment is administered at a dose of 2,5 mg/Kg (around 10 $\mu$g/injection) (group 2) by intraperitoneal injection once a week for a total of four weeks (Alleman et al., 2004). This dose is well below the limit of toxicity of the drug tolerated in a murine model (Momparler and Frith, 1981). Erythro-magneto-virosomes and erythro-magneto-virosomes encapsulating 5Aza-dC are administered by tail injection and concentrated at tumor mass by applying static magnetic field (Groups #3 and 4). $1 \times 10^5$ erythro-magneto-virosomes (group 3) and $1 \times 10^5$ erythro-magneto-virosomes containing 5-aza-dC (group 4) are tail injected with a total amount of 10$\mu$g/injection.

*Number of mice involved in the study*

Four groups of nude mice are involved in the study. One group of mice is used as a control and receives only the injection of saline solution (CTRL 1). This control group (Group #1) includes 12 mice each, divided into other 2 subgroups, which are sacrificed at four and six weeks following injections of control saline solution. The remaining three experimental groups (Groups #2,3 and 4) constituted of 12 mice each are treated with therapeutic compound such as 5-Aza-dC alone, with erythro-magneto-virosomes alone and erythro-magneto virosomes encapsulating therapeutic compound such as 5-Aza-dC (see table 1). Also these experimental groups are divided into other 2 subgroups, which are sacrificed at four and six weeks following treatments. The group #2 of mice receives intraperitonal injection of therapeutic compound as 5-AzadC at time 0. The group # 3 of mice receives tail injection of erythro-magneto-virosomes at time 0. The group # 4 of mice receive tail injection of erythro-magneto-virosomes encapsulating therapeutic compound as 5-Aza-dC at time 0.

These experimental groups include 12 mice per group, each divided into other 2 subgroups, that are sacrificed at four and 6 weeks following treatment (6 mice per subgroup X 2 sacrification times = total 12 mice per group; 4 experimental groups X 12 mice per group = 48 total mice for each set of experiment). Mice in each subgroup (constituted of 6 mice) are further divided in two other subgroups of 3 mice each (3 mice for fresh sample collection and 3 mice for paraffin embedded sample collection) in order to ensure there is enough material to perform the experiments. The experiments are repeated in the same exact condition if the first experiment is informative. Animals are housed in micro-isolator cages within climate-controlled laboratories. All mice are maintained under identical conditions of temperature (21 $\pm$ 1 °C), humidity (60 $\pm$ 5%) and light/dark cycle, and have free access to normal mice diet. The cages, food and water are inspected on a daily basis and replenished or changed on needed basis. Animals are monitored daily for signs of distress and pain by a certified veterinarian. For experiments involving administration of drugs, animals are anesthetized using isofluorane. Animals are sacrificed by $CO_2$ asphyxiation as recommended by the institutional guidelines. Animal weight is monitored every two days. Tumor volumes are calculated using the formula tumor volume = (length) x (width)$^2$/2. Alternatively, tumor size is calculated using a SonoSite ultrasound scanner. Tumor sizes of treated mice are compared to those of control mice. The tumors are then be excised and weighed before processing. Tissues that are used for molecular biological analysis are snap frozen in liquid nitrogen and stored at -80°C. Tissues to be sectioned are placed in OTC (Sakura Finetek USA, Inc., Torrance, CA), frozen in liquid nitrogen and stored at -80°C or preserved in neutral-buffered formalin at 4 °C before embedding in paraffin. The CD1 *nu/nu* mice are a suitable host for these studies. The use of animals is necessary because *in vitro* techniques cannot fully simulate the interactions between tumor cells and the tissue environment that plays a pivotal role in tumor growth. Experimental procedure and justification of the use of the mice are detailed in table 1.

All animal experiments in this project are performed at the Experimental Animal Core facility. The Experimental Animal Core houses its animals in micro-isolators in the Experimental Animal Facility adjacent to the research laboratories. An aseptic surgical area adjacent to the housing room is available for all procedures. The Animal Facility complies with all necessary national and Institutional animal care and Biosafety regulations.

### Tissue collection

The mice are sacrificed at 4 and 6 weeks post-treatment. Mice are divided in two groups for sample collection. The samples of the first group are perfused with 1 X PBS, and fixed with 10% formaldehyde. Tumors are then paraffin-embedded, cut into 5μm sections and stained with H&E. The tumor samples of the second group are collected, frozen in dry ice and kept at -80°C until use or are placed in OTC (Sakura Finetek USA, Inc., Torrance, CA), frozen in liquid nitrogen and stored at -80°C. Frozen tumor tissues are either used for western blot analysis and for RT-qPCR analysis. Tumor size, grade and stage, DNA ploidy and vascularization, that it is index of the progression of the tumor, are evaluated (Folberg et al., 1992). Statistic analysis are conducted through the use of Kaplan-Meier models. The coefficients of correlation will be calculated using the Spearman coefficient.

### Efficiency and efficacy of treatments

### Immuno-histochemical analysis.

Histological sections of the tumor mass are processed for immunohistochemistry following standard procedures. Formalin-fixed and paraffin-embedded samples are processed. Sections are mounted on glass and dried overnight at 37°C. All sections are de-waxed, rehydrated, quenched in 0.5% hydrogen peroxide and microwave pre-treated in 10 mM citrate buffer, pH 6.0. After blocking with normal serum for 1 hour at room temperature, tissue sections are incubated with polyclonal and monoclonal antibodies against tumor markers, either at room temperatures or at 4°C for the appropriate time. Negative controls are produced substituting the primary antibody with pre-immune serum. All slides are processed by the ABC method (Vector Laboratories, Burlingame, CA). Diaminobenzidine (DAB) is used as the final chromogen, and Gill's hematoxylin is used as a counterstaining.

### Electron Microscopy analysis

To evaluate the effective penetration of "erythro-magneto-virosomes" at cellular level, the tumor tissues from sacrificed animals are also fixed in 2,5% glutaraldehyde in 0.1 M cacodilate buffer for 2 hours at 4°C, post-fixed in 1% osmium in Veronal buffer, dehydrated and embedded in epoxidic resin. The sections are examined by a Zeiss EM 109 electron microscope.

### RNA preparation for RT-qPCR analysis

Total RNA samples are isolated from tumor masses dissected from the frozen tumor tissues using TRIZOL reagent (Invitrogen) according to the manufacturer's instructions. Concentration of purified RNA samples are determined by A260 measurement and the quality is checked by Lab-on-a-chip analysis (total RNA nano biosizing assay, Agilent) with the Agilent 2100 Bioanalyzer.

### Quantitative Real Time revere transcription-PCR.

To validate transcriptional responses in the series of treated animals, tumor biomarkers are evaluated by relatively RT-qPCR following standard procedures.

**RESULTS**

From the above experimental data it can be outlined that the viral spike glycoprotein (HA see Fig.2) purified from influenza virons (Fig.1) and reconstituted in erythro-magneto particles maintains its fusogenic properties with the target membrane as shown by the fusion assays in Fig. 4. The yield of therapeutic compound (exemplified by 5-Aza) reconstituted into erythro-magneto-virosomes is shown in fig. 5. The total amount of 5-Aza incapsulated in $1.10^6$ erythro-magneto virosomes correspond to $0.5 \mu M$. The uptake to the target cell and delivery of the targeted therapeutic compound is reported in Fig 6 and 7. The efficacy of the active drug (5-Aza) reconstituted in erythro-magneto virosomes to drive cell towards apoptosis is shown in the cell microscopy (fig.6 and 7) and in the FACS analysis (see fig.8a, b and c).

The results of the experiments indicate that the erythro-magneto virosomes drug delivery system increases the efficiency and efficacy of the therapeutic compound treatment. In fact, the total amount of the therapeutic compound necessary to obtain growth arrest and apoptotic response in tumor cells is six times less when erythro-magneto virosome delivery system is used when compared to standard therapy. Moreover, the anti-neoplastic effect of the exemplified therapeutic compound driven by the delivery system of the present invention appears 48 hrs earlier than when standard treatment is applied.

**Bibliographic references**

Pierigè F, et al., Adv Drug Deliv Rev. 2008 Jan 14;60(2):286-95

Rossi L, et al., Expert Opin Drug Deliv. 2005 Mar;2(2):311-22

J C Roth, et al., Cell vehicle targeting strategies. Gene Therapy. 2008; 15(10): 716-729.

Gutiérrez Millán C, et al., Transl Res. 2008; 152(2):59-66.

Gutiérrez Millán C, et al., J. Antimicrob Chemother. 2007; 61(2):375-81.

Yezhelyev MV, et al., Lancet Oncol. 2006;7(8):657-67

Alexiou C, Jurgons R, Seliger C, Iro H. J Nanosci Nanotechnol. 2006a; 6(9-10):2762-8.

Alexiou C, et al., Eur Biophys J. 2006b; 35(5):446-50.

Alexiou C, et al., Anticancer Res.2007; 27(4A):2019-22.

Davison and Sanford Infect Immun 32 (1) 1981

Folberg R et al., Human Pathos. 1992; 23(11): 1298-305

Alleman WG,et al. Clin Cancer Res. 2004; 10(20): 7011-21.

Momparler RL and Frith CH, Drug Chem Toxicol. 1981; 4(4):373-81.

**Claims**

1. A compound delivery system to a target cell comprising:

   - an haemoglobin-deprived erythrocyte,
   - a magnetic or super-paramagnetic nano-particle located inside the erythrocyte and
   - a protein located on the membrane of the erythrocyte able to induce the fusion of the membrane of said erythrocyte to the membrane of the target cell wherein the protein is a virosome-derived protein.

2. The system according to claim 1 wherein the magnetic or super-paramagnetic nano-particle is of $\gamma Fe_2O_3$.

3. The system according to claim 1 or 2 wherein the magnetic or super-paramagnetic nano-particle has a diameter between 5 to 100 nm.

4. The system according to any one of claim 1 to 3 wherein the magnetic or super-paramagnetic nano-particle is covered by a surfactant.

5. The system according to any one of preceding claims wherein the magnetic or super-paramagnetic nano-particle is further modified to comprise an additional agent.

6. The system according to claim 5 wherein the additional agent is selected from the group of an amino or carboxy or esterified group covalently bound to a fluorescent or non-fluorescent functional group.

7. The system according to any one of preceding claim wherein the virosome is from an influenza virus virosome or a vesicular stomatitis virus virosome or an Epstein Barr virus virosome.

8. The system according to any one of preceding claims being loaded with a pharmacological or biological therapeutic compound.

9. The system according to claim 8 wherein the pharmacological or biological therapeutic compound is selected from the group of: antiretroviral compound, small molecule, pro-drug, phosphorylated pro-drug or a chemiotherapeutic compound.

10. A system according to any one of preceding claims for use as a medicament.

11. The system according to claim 10 for use as a medicament wherein said medicament is administered via aerosol, parenteral or systemic route.

12. A method for the preparation of the system of claim 1 comprising:

a) depriving isolated erythrocyte of haemoglobin;
b) incubating under appropriated conditions the haemoglobin-deprived erythrocyte with a protein able to induce the fusion of the membrane of said erythrocyte to the membrane of a target cell wherein the protein is a virosome-derived protein, thus obtaining an erythrocyte-protein complex;
c) purifying said complex;
d) incubating the purified complex with magnetic or super-paramagnetic nano-particles under appropriated conditions.

13. A pharmaceutical composition comprising the system of any one of claims 1-9 and pharmaceutically acceptable diluents, carrier and/or excipients.


**Patentansprüche**

1. System zur Abgabe einer Verbindung an eine Targetzelle, umfassend:

- einen an Hämoglobin verarmten Erythrozyten,
- ein magnetisches oder superparamagnetisches Nanoteilchen, das sich innerhalb des Erythrozyten befindet und
- ein Protein, das sich an der Membran des Erythrozyten befindet und in der Lage ist, die Fusion der Membran des Erythrozyten an die Membran der Targetzelle zu induzieren, wobei das Protein ein von Virosom abgeleitetes Protein ist.

2. System nach Anspruch 1, wobei das magnetische oder superparamagnetische Nanoteilchen von $\gamma$-$Fe_2O_3$ stammt.

3. System nach Anspruch 1 oder 2, wobei das magnetische oder superparamagnetische Nanoteilchen einen Durchmesser zwischen 5 und 100 nm aufweist.

4. System nach einem der Ansprüche 1 bis 3, wobei das magnetische oder superparamagnetische Nanoteilchen durch ein Tensid bedeckt ist.

5. System nach einem der vorhergehenden Ansprüche, wobei das magnetische oder superparamagnetische Nanoteilchen noch weiter modifiziert ist, um ein zusätzliches Mittel zu umfassen.

6. System nach Anspruch 5, wobei das zusätzliche Mittel aus der Gruppe von einer Amino- oder Carboxy- oder veresterten Gruppe ausgewählt ist, die kovalent an eine fluoreszierende oder nichtfluoreszierende funktionelle Gruppe gebunden ist.

7. System nach irgendeinem vorhergehenden Anspruch, wobei das Virosom von einem Grippevirusvirosom oder einem vesikulären Stomatitisvirusvirosom oder einem Epstein-Barr-Visusvirosom stammt.

**8.** System nach irgendeinem von vorhergehenden Ansprüchen, das mit einer pharmakologischen oder biologischen therapeutischen Verbindung beladen ist.

**9.** System nach Anspruch 8, wobei die pharmakologische oder biologische therapeutische Verbindung aus der Gruppe ausgewählt ist von:

antiretroviraler Verbindung, Kleinmolekül, Pro-Drug, phosphoryliertem Pro-Drug oder einer chemischtherapeutischen Verbindung.

**10.** System nach einem der vorhergehenden Ansprüche zur Verwendung als Medikament.

**11.** System zur Verwendung als Medikament nach Anspruch 10, wobei das Medikament durch ein Aerosol, auf parenteralem oder systemischem Weg verabreicht wird.

**12.** Verfahren für die Herstellung des Systems nach Anspruch 1, umfassend:

a) Entziehen von Hämoglobin von isoliertem Erythrozyten;
b) Inkubieren des an Hämoglobin verarmten Erythrozyten unter geeigneten Bedingungen mit einem Protein, das in der Lage ist, die Fusion der Membran des Erythrozyten an die Membran einer Targetzelle zu induzieren, wobei das Protein ein von einem Virosom abgeleitetes Protein ist, wodurch ein Erythrozyten-Protein-Komplex erhalten wird;
c) Reinigen des Komplexes;
d) Inkubieren des gereinigten Komplexes mit magnetischen oder superparamagnetischen Nanoteilchen unter geeigneten Bedingungen.

**13.** Pharmazeutische Zusammensetzung umfassend das System nach einem der Ansprüche 1 bis 9 und pharmazeutisch akzeptable Verdünnungsmittel, Träger und/oder Trägerstoffe.

**Revendications**

**1.** Système de déliverance d'un composé à une cellule cible, comprenant:

- un érythrocyte privé d'hémoglobine,
- une nanoparticule magnétique ou super-paramagnétique située à l'intérieur de l'érythrocyte et
- une protéine située sur la membrane de l'érythrocyte capable d'induire la fusion de la membrane dudit érythrocyte à la membrane de la cellule cible, dans lequel la protéine est une protéine dérivée de virosome.

**2.** Système selon la revendication 1, dans lequel la nanoparticule magnétique ou super-paramagnétique est de $\gamma Fe_2O_3$.

**3.** Système selon la revendication 1 ou 2, dans lequel la nanoparticule magnétique ou super-paramagnétique a un diamètre entre 5 et 100 nm.

**4.** Système selon l'une quelconque des revendications 1 à 3, dans lequel la nanoparticule magnétique ou super-paramagnétique est recouverte d'un surfactant.

**5.** Système selon l'une quelconque des revendications précédentes, dans lequel la nanoparticule magnétique ou super-paramagnétique est en outre modifiée pour comprendre un agent supplémentaire.

**6.** Système selon la revendication 5, dans lequel l'agent supplémentaire est sélectionné dans le groupe d'un groupe amino ou carboxy ou estérifié lié de façon covalente à un groupe fonctionnel fluorescent ou non fluorescent.

**7.** Système selon l'une quelconque des revendications précédentes, dans lequel le virosome provient d'un virosome du virus de la grippe ou d'un virosome du virus de la stomatite vésiculaire ou d'un virosome du virus d'Epstein Barr.

**8.** Système selon l'une quelconque des revendications précédentes, étant chargé d'un composé thérapeutique pharmacologique ou biologique.

9. Système selon la revendication 8, dans lequel le composé thérapeutique pharmacologique ou biologique est sélectionné dans le groupe de : un composé antirétroviral, une petite molécule, un promédicament, un promédicament phosphorylé ou un composé chimiothérapeutique.

10. Système selon l'une quelconque des revendications précédentes, pour son utilisation en tant que médicament.

11. Système pour son utilisation en tant que médicament selon la revendication 10, dans lequel ledit médicament est administré par aérosol, par voie parentérale ou systémique.

12. Procédé de préparation du système selon la revendication 1, comprenant:

a) la privation en hémoglobine d'un érythrocyte isolé ;
b) l'incubation dans des conditions appropriées de l'érythrocyte privé d'hémoglobine avec une protéine capable d'induire la fusion de la membrane dudit érythrocyte à la membrane d'une cellule cible, dans lequel la protéine est une protéine dérivée de virosome, permettant ainsi d'obtenir un complexe érythrocyte-protéine ;
c) la purification dudit complexe ;
d) l'incubation du complexe purifié avec des nanoparticules magnétiques ou super-paramagnétiques dans des conditions appropriées.

13. Composition pharmaceutique comprenant le système selon l'une quelconque des revendications 1 à 9, et des diluants, un vecteur et/ou des excipients pharmaceutiquement acceptables.

B

A

Fig. 1

Fig. 2

Fig. 3

Fig. 4

Fig. 5

Fig. 6

Fig. 7

Fig. 8a

Fig. 8a

Fig. 8a

Fig. 8a

D

EP 2 376 125 B1

Fig. 8b

Fig. 8b

Fig. 8b

Fig. 8c

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2007004073 A

### Non-patent literature cited in the description

- **PIERIGÈ F et al.** *Adv Drug Deliv Rev.,* 14 January 2008, vol. 60 (2), 286-95
- **ROSSI L et al.** *Expert Opin Drug Deliv.,* March 2005, vol. 2 (2), 311-22
- **J C ROTH et al.** *Cell vehicle targeting strategies. Gene Therapy,* 2008, vol. 15 (10), 716-729
- **GUTIÉRREZ MILLÁN C et al.** *Transl Res.,* 2008, vol. 152 (2), 59-66
- **GUTIÉRREZ MILLÁN C et al.** *J. Antimicrob Chemother.,* 2007, vol. 61 (2), 375-81
- **YEZHELYEV MV et al.** *Lancet Oncol.,* 2006, vol. 7 (8), 657-67
- **ALEXIOU C ; JURGONS R ; SELIGER C ; IRO H.** *J Nanosci Nanotechnol.,* 2006, vol. 6 (9-10), 2762-8
- **ALEXIOU C et al.** *Eur Biophys J.,* 2006, vol. 35 (5), 446-50
- **ALEXIOU C et al.** *Anticancer Res.,* 2007, vol. 27 (4A), 2019-22
- **DAVISON ; SANFORD.** *Infect Immun,* 1981, vol. 32 (1
- **FOLBERG R et al.** *Human Pathos.,* 1992, vol. 23 (11), 1298-305
- **ALLEMAN WG.** *Clin Cancer Res.,* 2004, vol. 10 (20), 7011-21
- **MOMPARLER RL ; FRITH CH.** *Drug Chem Toxicol.,* 1981, vol. 4 (4), 373-81